# EUROPÄISCHE PATENTSCHRIFT

(11) **EP 2 596 757 B1**
(45) Veröffentlichungstag und Bekanntmachung des Hinweises auf die Patenterteilung: **16.07.2014**
(21) Anmeldenummer: 11190415.7
(22) Anmeldetag: 23.11.2011
(51) Int. Cl.: A61B 17/15, A61F 2/46, A61B 19/00

(54) **Vorrichtung für die Vorgabe einer Schnittebene für die Knochenresektion**
Device for setting a cut level for bone resection
Dispositif destiné à l'émission d'un plan de coupe pour la résection osseuse

(43) Veröffentlichungstag der Anmeldung: 29.05.2013
(73) Patentinhaber: Waldemar Link GmbH & Co. KG, 22339 Hamburg (DE)
(72) Erfinder: Dmuschewsky, Klaus, 22397 Hamburg (DE); Balzarini, Amos, 22844 Norderstedt (DE)
(74) Vertreter: Raffay & Fleck

(56) Entgegenhaltungen:
- EP-A1- 1 040 791
- US-A- 4 952 213
- US-A- 5 228 459
- US-A- 5 342 368
- US-A- 5 749 876

## Beschreibung

Die vorliegende Erfindung betrifft eine Vorrichtung für die Vorgabe einer Schnittebene für die Knochenresektion gemäß dem Oberbegriff des Anspruchs 1.

In der Medizin, insbesondere in der orthopädischen Chirurgie, werden für den Einsatz von Gelenkendoprothesen Resektionsschnitte zum Entfernen der verschlissenen Bereiche der an dem jeweiligen Gelenk beteiligten Knochen und zum Schaffen einer definierten Anbindungsgeometrie für die einzusetzenden Gelenkendoprothesenteile durchgeführt. Dabei kommt es ganz wesentlich darauf an, dass die Resektionsschnitte exakt geführt sind, so dass ein endoprothetischer Ersatz hinsichtlich der Geometrie, in der er im Körper des Patienten positioniert ist, eine optimale Funktion ermöglicht und den Patienten hinsichtlich einer Bewegungsfreiheit so wenig als möglich einschränkt. Dies gilt nicht nur, aber hier in besonderem Maße, im Bereich der Knieendoprothesen, wo bei der Resektion der distalen Femurkomponente sowie des proximalen Tibiaplateaus entsprechende Sorgfalt anzuwenden ist, um die postoperative Stellung und räumliche Lage des die Endoprothese tragenden Beines gegenüber der Ausgangssituation nicht, jedenfalls nicht in einer den Patienten beeinträchtigenden Weise zu verändern. Neben einer Beibehaltung der Beinlänge sind hierbei auch die korrekte Einstellung des Varus/Valgus-Winkels sowie des sogenannten Slopewinkels, also des Winkels der um eine Verbindungsachse beider Knie bei paralleler Beinstellung bzw. um eine parallel zu dieser Verbindungsachse verlaufende Achse gegenüber der Horizontalen verkippten Ebene, zu wählen. Sämtliche dieser Parameter werden - in Zusammenschau mit der Geometrie der jeweiligen Bestandteile der Endoprothese - durch die Lage der Schnittebenen der jeweiligen Knochenresektionen und damit die Lage der Anbindungsebenen für das an diese Schnittebene anzusetzende Teil der Endoprothese bestimmt.

Eine exakte Einstellung der Schnittebene hinsichtlich eines längs verschieblichen Abstandes sowie hinsichtlich wenigstens einer Verkippungsebene, ist aber auch bei anderen Knochenresektionen wichtig, beispielsweise bei der Resektion des Humeruskopfes im Falle der operativen Versorgung mit einer Schulterendogelenkprothese.

Entsprechend werden bei derartigen Operationen die Knochenresektionsschnitte nicht frei Hand geführt, sondern die Position und Lage der Schnittebene wird mit entsprechenden Hilfsvorrichtungen am freigelegten Knochen zunächst geplant, im Anschluss wird die Resektion mit einem in der Schnittführungsstruktur einer solchen Vorrichtung geführten Schneidwerkzeug vollzogen.

Derartige Vorrichtungen umfassen einen an dem Knochen positionierbaren Basiskörper und einen Schnittführungskörper, an dem die Schnittführungsstruktur ausgebildet ist. Basiskörper und Schnittführungskörper können dabei zur gezielten Einstellung der Schnittebene relativ zueinander verlagert werden, insbesondere um wenigstens eine erste Achse zum Verkippen der Schnittebene verkippt werden. Häufig wird unter der Operation zunächst der Basiskörper am Knochen fixiert, z.B. mittels Fixierungspins, die in in den Knochen eingebrachte Bohrungen eingesetzt werden, und es wird dann durch entsprechendes Verstellen bzw. Positionsverlagern des Schnittführungskörpers die Schnittführungsstruktur zum Anbringen der gewünschten Resektion in der passenden Schnittebene eingestellt. Nun kann auch dieser Schnittführungskörper bei vielen bekannten Vorrichtungen am Knochen festgelegt werden, beispielsweise wiederum mittels in in den Knochen eingebrachte Bohrungen eingeführter und dort festgelegter Befestigungspins. Eine entsprechende Vorrichtung, die speziell für die Vorgabe einer Schnittebene bei der Tibiaresektion eingerichtet und gedacht ist, ist in der EP 1 444 956 A1 beschrieben. Entsprechende Vorrichtungen mit Verwendbarkeit für die Knochenresektion des proximalen Tibiaplateaus wie auch des distalen Femurplateaus sind in der EP 1 269 924 A1 bzw. in der EP 1 444 957 A1 beschrieben. Die Vorrichtung in der letztgenannten Druckschrift zeigt eine solche, bei der ausgehend von einem an dem Knochen festgelegten Basiskörper ein daran über verschiedene Lagerböcke angeordneter Schnittführungskörper sowohl für die Einstellung eines Varus/Valgus-Winkels um eine erste Ebene verkippt werden kann, als auch zur Einstellung des sogenannten Slopewinkels um eine zweite, zu der ersten Achse senkrechte Achse verkippt werden kann und schließlich auch hinsichtlich der Höhe, also des Abstandes zu dem Basiskörper einstellbar ist. Insbesondere im Hinblick auf die Verwendung für die Resektion des proximalen Tibiaplateaus besitzt die dort offenbarte Lösung einen erheblichen Vorteil gegenüber der Lösung, wie sie beispielsweise in der EP 1 040 791 A1 offenbart ist und bei der die Einstellung jedenfalls eines der genannten Winkel, insbesondere des Slope-Winkels durch Einstellen der Rastposition einer Führungs- und Haltestange im Bereich einer am Fuß des Patienten bei seinem Sprunggelenk festzulegenden Fußklammer einzustellen ist. Denn eine feste Festlegung der Fußklammer dort ist in der Regel schwerlich möglich, da keine übermäßig großen Haltekräfte aufgebracht werden können, um die Blutversorgung des Fußes nicht zu gefährden. Entsprechend ist es häufig nicht einfach, diese Fußklammer stabil zu halten und damit durch Justage in diesem Bereich eine entsprechende genaue und feine Einstellung der Winkelpositionen durchzuführen. Dies ist, wie bereits ausgeführt, bei der Lösung gemäß der EP 1 444 971 A1 besser gelöst, da die Einstellung sämtlicher Parameter für die Vorgabe der Lage der Schnittebene in einem Bereich vorgenommen wird, der unmittelbar an den Ort des zu setzenden Resektionsschnittes angrenzt, also über einen an dem zu resektionierenden Knochen selbst festzulegenden Basiskörper.

Allerdings ist insbesondere hinsichtlich der Verstellung der Verkippung(en) der Schnittebene(n) hinsichtlich des Slopewinkels bzw. des Varus/Valgus die in dieser Druckschrift offenbarte Vorrichtung vergleichsweise bedienerunfreundlich. Darüber hinaus werden zum Fixieren der Winkelpositionen der Schnittebene Schrauben eingesetzt, die sich insbesondere beim Auftreten von Vibrationen, wie sie durch ein für die Durchführung der Knochenresektion eingesetztes Schneidwerkzeug erzeugt und über den als Schnittführungsstruktur vorgesehenen Schnittführungsschlitz auf den Schnittführungskörper übertragen werden, lösen können. Dadurch besteht die Gefahr einer Verstellung bzw. Verlagerung der voreingestellten Schnittebene während des Resektionsschnittes, so dass schlimmstenfalls eine Nachresektion erforderlich wird.

Die US 5 342 368 offenbart eine sehr komplex aufgebaute Kulissenführung.

Hier soll mit der Erfindung Abhilfe geschaffen werden, indem eine Vorrichtung der eingangs bezeichneten Art und mit den Merkmalen des Oberbegriffs des Anspruchs 1 dahingehend weitergebildet wird, dass der Kippmechanismus einfach zu bedienen und hinsichtlich der Positionstreue der einmal in ihrer Lage eingestellten Schnittebene auch bei Durchführung des Resektionsschnittes zuverlässig ist.

Eine Lösung dieser Aufgabe wird mit der Erfindung geboten durch eine Vorrichtung mit den Merkmalen des Patentanspruchs 1. Vorteilhafte Weiterbildungen der erfindungsgemäßen Vorrichtung sind in den abhängigen Ansprüchen 2 bis 11 angegeben.

Die erfindungsgemäße Vorrichtung zeichnet sich also aus durch einen besonders gestalteten Kippmechanismus. Dieser weist Bestandteile an zwei relativ zueinander um die erste Achse verkippbaren Elementen auf. Bei diesen Elementen kann es sich unmittelbar um den Basiskörper und/oder den Schnittführungskörper handeln.Diese Elemente können aber auch zwischen diesen Basiskörper und Schnittführungskörper angeordnete, für die Verwirklichung einer Bewegbarkeit um mehrere Freiheitsgrade vorgesehene Lagerböcke sein bzw. enthalten. Derartige Lagerböcke können mithin Bestandteil der erfindungsgemäßen Vorrichtung sein, müssen dies jedoch nicht.

An einem ersten der Elemente, an denen Bestandteile des Kippmechanismus angeordnet sind, befinden sich erfindungsgemäß eine zu dem Kippmechanismus gehörende geradlinige Führung und ein entlang dieser Führung in deren Längsrichtung linear verschiebbarer Schieber. An dem Schieber ist ein Wirkabschnitt gebildet, der beim Bewegen desselben gegenüber einem an dem zweiten Element ausgebildeten und zu dem Kippmechanismus gehörigen Führungsabschnitt liegt, z.B. unter Angreifen an demselben an diesem entlang gleitet (aber auch mit einem Abstand gegenüber diesem sich bewegen kann). In dem Führungsabschnitt und dem Wirkabschnitt sind miteinander zusammenwirkende Strukturen ausgebildet, die eine quer zu der Längsrichtung der Führung wirkende Kulissenführung ausbilden. Mit dieser Kulissenführung wird als Reaktion auf eine lineare Verschiebung des Schiebers entlang der Führung eine Verkippung des ersten Elementes relativ zu dem zweiten Element um die erste Achse bewirkt. Hierzu können mit Vorteil das erste Element und das zweite Element entsprechend miteinander verbunden sein über eine gelenkige, die erste Achse definierende Verbindung.

Die Ausgestaltung des Kippmechanismus mit einem entlang der Führung längs verschiebbaren Schieber und einer durch diesen betätigten Kulissenführung ist zum einen besonders einfach zu bedienen. Zum anderen ist, insbesondere, aber nicht nur wenn der Schieber mit einer quer zu der Längsrichtung der Führung wirkenden Kraft an der Führung in einer gewünschten Position festgelegt wird, ein beispielsweise durch Vibrationen eines Schneidgerätes hervorgerufenes unabsichtliches Lösen des Schiebers aus der angewählten Position nahezu unmöglich. Dies ergibt sich aus der Winkeligkeit der jeweiligen Bewegungsrichtungen. Denn hierzu müsste der Schieber in Längsrichtung der Führung unter Aufbringung einer quer dazu wirkenden Kraft auf die Kulissenführung verschoben werden, was ein für ein unabsichtliches Verschieben ungünstiges Kräfteverhältnis ist. Im Übrigen können mit der erfindungsgemäßen Umsetzung die Dimensionierung der einzelnen Elemente und ihre Lage in Bezug auf die Drehachse so gewählt werden, dass durch einen vergleichsweise großen Abstand des Schiebers von der Achse, um die die Verkippung erfolgt, verglichen zu einem vergleichsweise geringen Abstand der Schnittführungsstruktur zu dieser Achse ein solches Kräfteverhältnis eingestellt ist, das zum Verkippen des einen Elementes relativ zu dem anderen auf den Schnittführungskörper, genauer die Schnittführungsstruktur eine erheblich größere Kraft aufzuwenden ist als auf den Schieber. Dadurch ergibt sich eine weitere Absicherung des Haltens der einmal mit dem Schieber eingestellten Kippposition.

Die Kulissenführung ist so ausgebildet wie in Anspruch 1 beschrieben. Demnach ist in dem Führungsabschnitt eine gegenüber der Längsrichtung gekrümmte Führungsnut und an dem Wirkabschnitt des Schiebers ein in dieser geführter Führungsstift bzw. Führungszapfen ausgebildet. Führungsnut bzw. Führungsschlitz einerseits und Führungsstift bzw. Führungszapfen andererseits bilden im Zusammenwirken dann die Kulissenführung.

Wie bereits erwähnt kann, zum Halten der einmal eingestellten Winkelposition der Schieber in mindestens zwei unterschiedlichen Positionen der Führung verrastbar sein, wozu an dem Schieber entsprechende Rastmittel und an der Führung mit diesen zusammenwirkende Raststrukturen ausgebildet sind. Dabei sind Rastmittel und Raststrukturen bevorzugt derart ausgebildet, dass sich eine Vielzahl von Rastpositionen ergeben, in denen der Schieber an unterschiedlichen Längspositionen entlang der Längsrichtung der Führung verrastet werden kann.

Gemäß einer weiteren vorteilhaften Ausgestaltung der Erfindung ist der Schieber von der geradlinigen Führung lös- und abnehmbar an dieser angeordnet. Dies ist insbesondere, da die erfindungsgemäße Vorrichtung für wiederholte Verwendungen vorgesehen ist, für die Reinigung und Sterilisation nach durchgeführter Operation von Vorteil. Aus gleichem Grund wird bevorzugt, wenn bei der erfindungsgemäßen Vorrichtung die einzelnen relativ zueinander beweglichen Teile lösbar miteinander verbunden sind und für Reinigungs- und Sterilisationszwecke einfach getrennt werden können. Dies kann im Hinblick auf gegeneinander verkippbare Elemente beispielsweise dadurch geschehen, dass entsprechende Schwenkachsen mit Abflachungen versehen und die Achsführungen mit einem entsprechend dem Durchmesser der Achse über die Abflachungen versehenen Öffnungsschlitz ausgebildet sind. Durch Verkippen der beiden Elemente derart gegeneinander, dass die Abflachungen mit der Öffnung fluchten, lassen sich solche Elemente dann einfach voneinander trennen. Die Trennung des Schiebers von der geradlinigen Führung bzw. der anderen relativ zueinander beweglichen Teile voneinander ist mit Vorteil besonders einfach und mit wenigen einfachen Handgriffen möglich.

Eine einfache und robuste Ausgestaltung von Führung und Schieber ergibt sich, wenn die gradlinige Führung gemäß Anspruch 4 eine im Querschnitt rechteckige, insbesondere quadratische, Führungsschiene ist und der Schieber hülsenartig ausgebildet ist mit einer der Querschnittskontur der Führungsschiene entsprechenden, von einer Wandung bis auf eine durchgehende schlitzförmige Öffnung umschlossenen Innenkontur, wobei durch Wahl des Materials und der Wandstärke die schlitzförmige Öffnung entgegen einer durch das Material ausgeübten Federkraft spreizbar ist. Durch diese Ausgestaltung kann insbesondere bei entsprechend ausgewählter Dimensiohierung der Führungsschiene sowie der Innenkontur des Schiebers ein Verklemmen bzw. Verrasten des auf die Führungsschiene aufgeschobenen Schiebers allein aufgrund der durch das Material aufgebrachten Federkraft erreicht werden. Hier sind also nicht etwa weitere Federelemente oder dgl. kleinteilige Strukturen erforderlich, was einesteils die Gesamtzahl der verbauten Teile klein und damit die Herstellungskosten gering hält, zum anderen aber insbesondere hinsichtlich der Reinigung und Sterilisation nach der Operation Vorteile mit sich bringt und auch die Gefahr eines etwaigen Verlustes von Kleinteilen im Operationsumfeld, im schlimmsten Fall des Verlierens eines solchen Teiles und Belassens in der Wunde, ausschließt. Für die Rastwirkung können auf der Innenseite des Schiebers beispielsweise entsprechende Vorsprünge, z.B. Stege, ausgebildet sein, die in entlang der Schiene ausgebildeten Rastvertiefungen eingreifen können. Um ein einfaches Verschieben des Schiebers zu ermöglichen, können die Flanken derartiger Rastvorsprünge oder -stege angeschrägt oder mit sonstiger Kantenführung gleitend übergehend gebildet sein, so dass sie über entsprechend angeschrägte Begrenzungen der Rastvertiefungen hinweg gleiten können durch Aufbringen einer diese Haltekraft übersteigenden Kraft.

Weiterhin ist von Vorteil, wenn entlang der gradlinigen Führung eine Winkelskala angebracht ist und auf dem Schieber ein mit der Winkelskala zum Ablesen zusammen bringbarer Zeiger. So kann für eine bestimmte Position des Schiebers in der Längsrichtung durch die Zeigerstellung relativ zu der Winkelskala eine Winkelstellung der dadurch um die erste Achse verkippten Schnittebene relativ zu einem vorgegebenen Referenzwinkel abgelesen werden. Eine solche Winkelskala kann beispielsweise helfen, einen präoperativ z.B. durch bildgebende Verfahren bestimmten, für die Winkeleinstellung der Schnittebene bei Verkippung um die Achse (z.B. eine Varus/Valgus-Winkelseinstellung oder eine Slope-Winkeleinstellung im Falle einer für das Einsetzen einer Knieendogelenkprothese durchzuführenden Resektion am distalen Femurplateau oder am proximalen Tibiaplateau) Winkel voreinzustellen. Auch ist es möglich einen durch Augenmaß des Operateurs eingestellten Winkel abzulesen und mit entsprechend vorherbestimmten Daten zu vergleichen, um hier eine zusätzliche Sicherheit und Referenz zu gewinnen.

Für eine einfache Bedienbarkeit des bei der erfindungsgemäßen Vorrichtung vorgesehenen Kippmechanismus ist es von Vorteil, wenn die Kulissenführung derart gestaltet ist, dass sich eine lineare Umsetzung der Wegstrecke, um die der Schieber verlagert wird, zu einem Verkippungswinkel, um den die Schnittebene um die erste Achse verkippt wird, ergibt. Mit anderen Worten wird für die Verkippung um ein weiteres Grad eine ebenso lange Wegstrecke beim Verschieben des Schiebers entlang der gradlinigen Führung benötigt, wie für das Kippen eines solchen Grades zuvor oder im Anschluss. Diese lineare Umsetzung gibt dem Operateur ein besseres Gefühl für die Einstellung eines Winkels, sofern er hier nach einem bestimmten Winkelwert arbeiten oder zunächst eine erste Voreinstellung vornehmen will.

Insbesondere dort, wo mit einer erfindungsgemäßen Vorrichtung nicht nur eine Verkippung der Schnittebene um eine einzige Achse, sondern um zwei verschiedene Achsen vorzunehmen ist, beispielsweise eine Einstellung des Varus/Valgus-Winkels einerseits und des Slopewinkels andererseits bei der Einstellung der Schnittebene für die Resektion am proximalen Tibiaplateau, kann mit Vorteil für jede der vorzunehmenden Verkippungen und möglichen Einstellungen ein entsprechender Kippmechanismus wie oben beschrieben vorgesehen sein, also ein erster und ein zweiter Kippmechanismus, die beide über eine gradlinige Führung und einen Schieber, die zusammen eine Kulissenführung in der oben beschriebenen Weise bilden, verfügen. Dabei sind zweckmäßigerweise die beiden Kippmechanismen unabhängig voneinander zu betätigen. Dies wird typischerweise dadurch gelingen, dass zwischen dem Basiskörper und dem Schnittführungskörper noch wenigstens ein Lagerbock angeordnet wird, wobei dieser Lagerbock relativ zu dem Basiskörper um eine erste Achse verkippbar ausgebildet und an dem Basiskörper gelagert ist, der Schnittführungskörper an dem Lagerbock entsprechend um eine zweite, von der ersten Achse verschiedene und zu dieser nicht parallele, insbesondere zu dieser senkrechteAchse verkippbar angeordnet ist. Mit anderen Worten sind hier also bei einer solchen Verkörperung der erfindungsgemäßen Vorrichtung zwei gleich aufgebaute Kippmechanismen mit jeweils einem entlang einer gradlinigen Führung verschiebbaren Schieber und einer durch Zusammenwirkung eines Wirkabschnittes des Schiebers mit einem Führungsabschnitt ausgebildeten Kulissenführung vorhanden, wobei hier jeweils beide Schieber bzw. Kulissenführungen wie vorstehend näher ausgeführt ausgebildet sind.

Um mit der erfindungsgemäßen Vorrichtung auch hinsichtlich eines weiteren Freiheitsgrades eine Einstellung der Lage der Schnittebene vornehmen zu können, kann eine Höhenverstelleinrichtung vorgesehen sein, mittels derer der Schnittführungskörper relativ zu dem Basiskörper in einer Höhenrichtung linear verlagerbar und in seinem Abstand zu diesem festlegbar ist. Dies kann z.B. durch Zwischenschalten eines weiteren Lagerbockes geschehen, der gegenüber dem Basiskörper höhenverstellbar ist und an dem ein weiterer Lagerbock oder der Schnittführungskörper um eine Achse verschwenkbar festliegt. Diese Höhenverstelleinrichtung kann mit Vorteil eine Stellschraube zum Einstellen des Abstandes zwischen Basiskörper und Schnittführungskörper aufweisen. Diese Stellschraube kann insbesondere eine solche mit geringer Gewindesteigung sein, um eine Feinjustage der Höhenverstellung bzw. Höheneinstellung vornehmen zu können. Auch hier kann eine Skala vorgesehen sein, anhand derer der Operateur einen Abstandswert beispielsweise zwischen Basiskörper und Schnittführungskörper ablesen kann.

Die erfindungsgemäße Vorrichtung kann dabei insbesondere als eine solche für die Knochenresektion am proximalen Tibiaplateau oder aber eine solche für die Knochenresektion am distalen Femurplateau eingerichtet sein.

Im Falle einer Einrichtung der Vorrichtung für die Knochenresektion am proximalen Tibiaplateau kann diese mit Mitteln zur Anbindung an eine mit einer Fußklammer zu verbindende extramedulläre Ausrichtstange versehen sein, mittels derer der Basiskörper in einer ersten groben Ausrichtung positioniert werden kann. Mit Vorteil ist mit derselben Vorrichtung auch die Verbindung derselben mit einer intramedullär gesetzten Referenz, z.B. einer dort angeordneten Ausrichtstange möglich, um somit dem mit dieser Vorrichtung arbeitenden Operateur eine große Varietät der Ausrichtungsmöglichkeiten zu geben und ihm ein Arbeiten in der von ihm bevorzugten und ihm vertrauten Methode zu ermöglichen. Auch kann mit Vorteil bei der Realisierung für die Knochenresektion am proximalen Tibiaplateau die erfindungsgemäße Vorrichtung eine entsprechende Aufnahme für eine Peilstange enthalten, die eine senkrecht zu dem eingestellten Varus/Valgus-Winkel sich erstreckende Aufnahme der Peilstange ermöglicht, um somit einem Operateur eine Ausrichtung hinsichtlich einer Peilung in Richtung des zugehörigen Fußes bzw. Sprunggelenkes zu erlauben.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: eine Darstellung eines ersten Ausführungsbeispiels einer erfindungsgemäßen Vorrichtung für die Vorgabe einer Schnittebene für die Knochenresektion, hier für die Knochenresektion am oberen Tibiaplateau in schematischer Anordnung an einer Tibia;
- Fig. 2: die Vorrichtung gemäß dem Ausführungsbeispiel aus Fig. 1 in ähnlicher Anordnung aus anderer Perspektive gesehen;
- Fig. 3: in Explosionsdarstellung Teile der Vorrichtung gemäß dem Ausführungsbeispiel aus Figuren 1 und 2;
- Fig. 4: weitere Elemente des Ausführungsbeispiels der Vorrichtung gemäß Figuren 1 und 2 in einer Explosionsdarstellung;
- Fig. 5: den oberen Abschnitt der Vorrichtung gemäß dem Ausführungsbeispiel aus Fig. 1 in zusammengesetzter Form, allerdings ohne angesetzten Taster für die Kondyle;
- Fig. 6: eine Aufsicht auf den oberen Bereich des Ausführungsbeispiels gemäß Fig. 1 in einer unverkippten 0°-Stellung des Varus/Valgus-Winkels;
- Fig. 7: eine der Fig. 6 vergleichbare Ansicht, jedoch mit einem von 0° abweichend eingestellten Varus/Valgus-Winkel;
- Fig. 8: eine Darstellung des in Fig. 5 dargestellten oberen Abschnittes des ersten Ausführungsbeispiels für eine erfindungsgemäße Vorrichtung mit einer bezüglich des Slope-Winkels gewählten 0°-Einstellung;
- Fig. 9: eine der Fig. 8 vergleichbare Ansicht des oberen Abschnittes der Vorrichtung gemäß dem ersten Ausführungsbeispiel mit von 0° abweichend eingestelltem Slope-Winkel;
- Fig. 10: ein zweites Ausführungsbeispiel einer erfindungsgemäßen Vorrichtung für die Vorgabe einer Schnittebene für die Knochenresektion, hier für die Resektion am unteren Femurplateau, ohne aufgesetztem Schnittführungsblock mit der Schnittführungsstruktur;
- Fig. 11: eine Aufsicht auf die Vorrichtung gemäß dem zweiten Ausführungsbeispiel in einer bezüglich das Varus/Valgus-Winkels gewählten 0°-Stellung;
- Fig. 12: eine Aufsicht auf das zweite Ausführungsbeispiel mit von 0° abweichend eingestelltem Varus/Valgus-Winkel;
- Fig. 13: eine perspektivische Ansicht des zweiten Ausführungsbeispiels;und
- Fig. 14: eine perspektivische Ansicht des zweiten Ausführungsbeispiels in einer Anordnung an einem Femur und mit aufgesetztem Schnittführungsblock.

Die Darstellungen in den Figuren sind schematisch und nicht zwingend maßstabsgerecht.

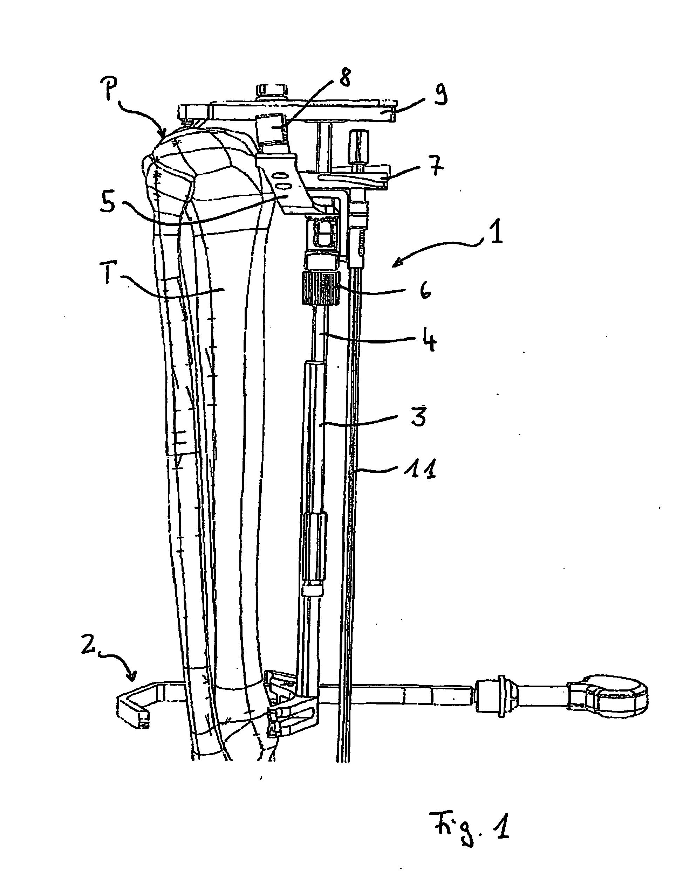

Zur Beschreibung der gezeigten Ausführungsbeispiele wird zunächst unter Bezugnahme auf die Figuren 1 bis 9 ein erstes Ausführungsbeispiel der Erfindung beschrieben, welches eine Vorrichtung für die einstellbare Vorgabe der Schnittebene des Resektionsschnittes am oberen Tibiaplateau zeigt.

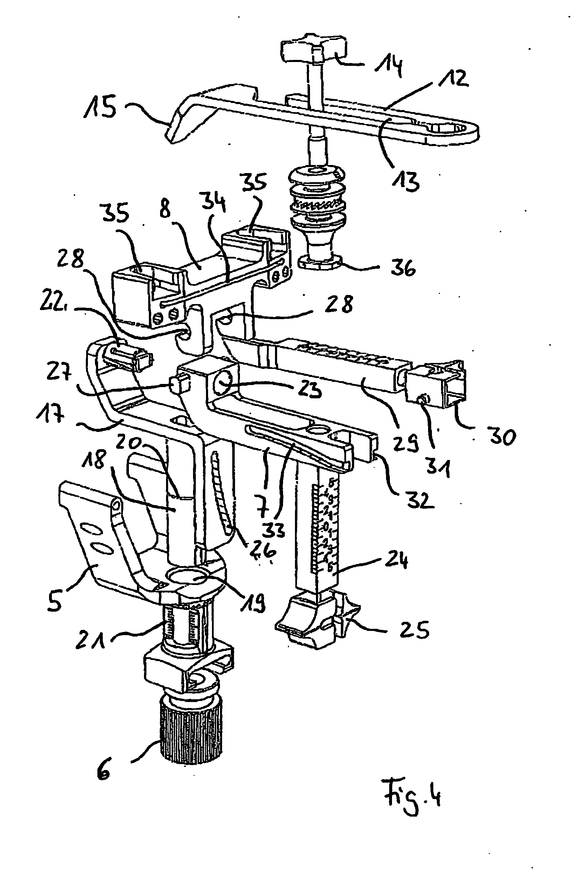

Unter Bezugnahme auf Fig. 1 ist dort eine solche Vorrichtung für die Vorgabe einer Schnittebene für die Tibiaresektion in einer schematischen Anordnung an einem Unterschenkelknochen, insbesondere dessen Tibia T gezeigt und als Vorrichtung allgemein mit 1 bezeichnet. Die Vorrichtung 1, die in der tatsächlichen Verwendung selbstverständlich nicht an einer vollständig freigelegten Tibia T, sondern vielmehr an einem Unterschenkel eines Beines, bei dem lediglich der zu operierende Bereich des Knies freigelegt ist, angeordnet wird, enthält eine Fußklammer 2, mit der sie mit einem unteren Abschnitt im Bereich des Knöchels am Bein eines Patienten festgelegt werden kann. Ausgehend von der Fußklammer 2 und fest an dieser befestigt erstreckt sich ein Aufnahmerohr 3, in welchem teleskopartig eine Ausrichtstange 4 aufgenommen und linear geführt ist. Mit der Ausrichtstange 4 verbunden ist ein jochförmiger Basiskörper 5, an dem wiederum ein erster Lagerbock 17 (vgl. Fig. 4) angeordnet ist, der gegenüber dem Basiskörper 5 über eine Schraubhülse 6 zur Feinjustage einer Höheneinstellung in Längsrichtung der Ausrichtstange 4 bewegbar und in einer Position einstellbar ist.

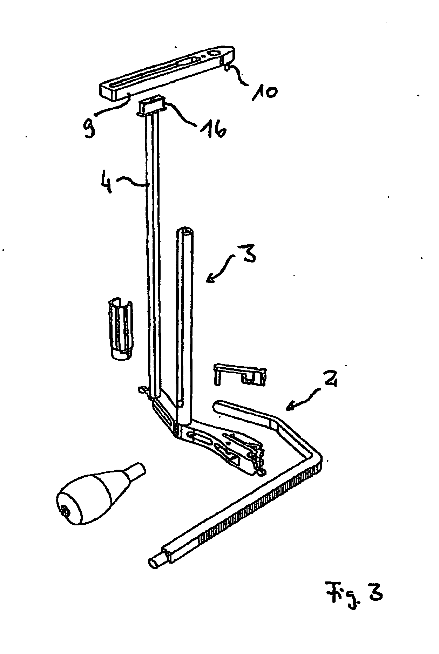

An dem ersten Lagerbock 17 ist gegenüber diesem zur Einstellung eines Varus/Valgus-Winkels in nachfolgend noch näher erläuterter Weise verdrehbar ein zweiter Lagerbock 7 angelagert. An dem Lagerbock 7 wiederum ist - erneut um eine Achse verdrehbar, diesmal zur Einstellung des Slope-Winkels - ein Schnittführungsblock 8 angelagert. Ferner ist ein am oberen Ende der Ausrichtstange 4 angeordneter Klemmbügel 9 zu erkennen, der an seinem auf dem Plateau P der Tibia T aufliegenden Abschnitt einen hier nicht näher zu erkennenden, aber insbesondere in Fig. 3 sichtbaren Dorn 10 aufweist, mit welchem er in das Tibiaplateau eingeschlagen werden kann. Somit gibt im Zuge einer ersten Voreinstellung der erfindungsgemäßen Vorrichtung 1 am unteren Abschnitt der Tibia T die Flussklammer 2, am oberen Abschnitt der Tibia T der Klemmbügel 9 einen ersten Halt, insbesondere eine seitliche Fixierung.

Zu erkennen ist ferner ein in den Lagerbock 7 eingehängter Peilstab 11, der als Peilhilfe für die Einstellung des Varus/Valgus-Winkels dient.

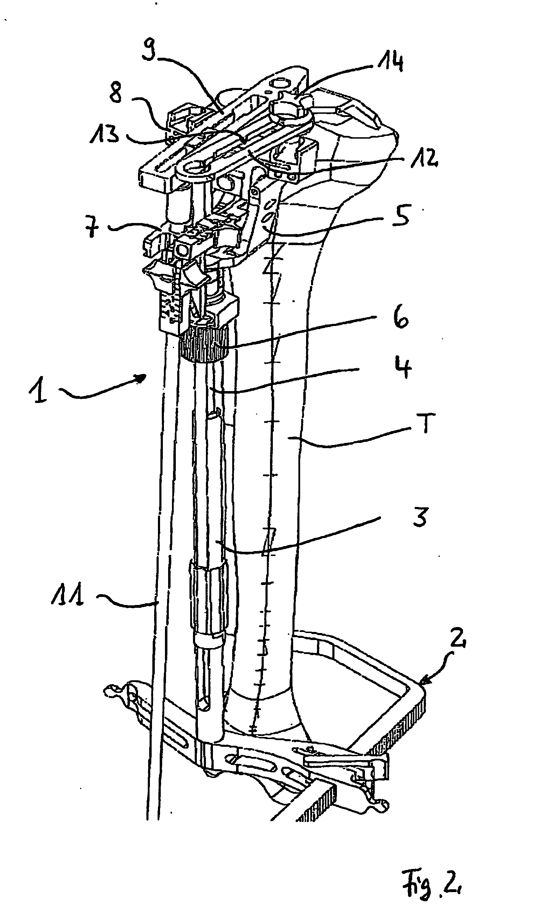

In Fig. 2 ist die in Fig. 1 dargestellte Vorrichtung noch einmal aus anderer Perspektive gezeigt, wobei hier noch ein an dem Schnittführungsblock 8 festgelegter Kondylentaster 12 gezeigt ist, der lösbar an dem Schnittführungsblock 8 festgelegt werden kann über einen im Kondylentaster 12 ausgebildeten Längsschlitz 13, durch den hindurch eine Verbindungsschraube 14 geführt ist, in einer Längsposition verschoben werden kann. Der Kondylentaster 12 hat dabei, wie insbesondere in Fig. 4 zu erkennen ist, ein gekröpftes Vorderende 15, welches die Tastspitze des Kondylentasters 12 bildet. In der Anwendung wird diese Tastspitze auf einen tiefsten Punkt der verschliessenen Kondyle aufgesetzt, um von hier aus die Schnitthöhe des Resektionsschnittes am Tibiaplateau zu bestimmen.

In Fig. 3 sind einzelne Elemente der Fußklammer 2 mit dem daran angeordneten Aufnahmerohr 3 sowie die Ausrichtstange 4 und der lösbar an der Ausrichtstange 4 anzuordnende Klemmbügel 9 gezeigt. Zu erkennen ist dabei, dass der Klemmbügel 9 durch einfaches Aufsetzen auf ein T-förmiges Verbindungsstück 16 am oberen Ende der Ausrichtstange 4 einfach und lösbar an diesem oberen Ende der Ausrichtstange 4 festgelegt werden kann.

In Fig. 4 sind die Komponenten der Vorrichtung 1, die an deren oberen Ende angeordnet sind, noch einmal in einer Explosionsdarstellung gezeigt. Diese Vergrößerung dient insbesondere auch der Veranschaulichung der erfindungsgemäßen Wirkweise der Einstellmittel zum Verstellen der für die Vorgabe der Schnittebene zu wählenden Varus/Valgus-Winkel und/oder Slope-Winkel.

Gut zu erkennen ist hier, dass sich dieser obere Teil der erfindungsgmäßen Vorrichtung aus vier Hauptkomponenten, dem jochartigen Basiskörper 5, dem an diesem angesetzten ersten Lagerbock 17, dem zweiten Lagerbock 7 und dem Schnittführungsblock 8 zusammensetzt. Der erste Lagerbock 17 weist einen Hohlzapfen 18 auf, mit dem er verdrehfest in einer Zapfenaufnahme 19 am Basiskörper 5 angeordnet ist. Über die Schraubhülse 6 kann der Hohlzapfen 18 in der Zapfenaufnahme 19 in deren Längsrichtung auf und ab bewegt werden zur Feineinstellung der Resektionsschnitthöhe, die durch den entsprechenden Längsabstand zwischen dem Basiskörper 5 und dem ersten Lagerbock 17 bestimmt wird. Um hier eine Vereinfachung der Überprüfung einer Einstellung zu gewähren, ist an dem Hohlzapfen 18 ein Peilstrich 20 als Markierung angeordnet, der durch ein Sichtfenster in der Zapfenaufnahme 19 erkennbar wird und mit einer Skala 21 zusammenfällt, an der eine Positionierung bzw. Längsverschiebung des Lagerbocks 17 gegenüber der Basis 5, z.B. nach Millimetern abgelesen werden kann.

An dem Lagerbock 17 ist ein Drehzapfen 22 angeordnet, dessen Ausrichtung senkrecht zur Längsrichtung des Hohlzapfens 18 verläuft. Auf diesem Drehzapfen 22 sitzt der Lagerbock 7 mit einem Zapfenloch 23 und ist somit um eine durch die Längsachse des Drehzapfens 22 definierte Drehachse gegenüber dem Lagerbock 17 relativ verdrehbar. Zur Einstellung eines diesbezüglichen Winkels, der den Varus/Valgus-Winkel der Schnittebene wiedergibt, ist an dem Lagerbock 7 eine lineare Führungsbahn 24 angeordnet, die sich im zusammengefügten Zustand in einer Normalstellung in etwa entlang der Längsrichtung des Hohlzapfens 18 erstreckt. Auf diese im Querschnitt rechteckige lineare Führungsbahn 24 ist ein Schieber 25 aufgeschoben, der einen in etwa G-förmigen Querschnitt aufweist, also auf seiner Oberseite einen Öffnungsschlitz hat. Rückwärtig an diesem Schieber 25 ist, hier nicht zu erkennen, ein Führungsstift angeformt, der in eine Führungsnut 26 an dem Lagerbock 17 eingreift. Diese Führungsnut 26 verläuft im Vergleich zur Längsachse des Hohlzapfens 18 geneigt entlang einer gekrümmten Bahn. Im Zusammenwirken mit dem an dem Schieber 25 angeordneten Zapfen bildet diese Führungsnut 26 eine Kulissenführung, die bewirkt, dass bei einer Längsverschiebung des Schiebers 25 entlang der linearen Führungsbahn 24 eine Rotation des Lagerbocks 7 um den Drehzapfen 22 am Lagerbock 17 erfolgt. Der Schieber 25 ist in seiner Dimensionierung und in seinem Verhältnis zu der Außenform der linearen Führungsbahn 24 so gebildet, dass er entlang dieser verschoben werden kann, jedoch aufgrund seiner durch das Material selbst aufgebrachten Klemmkräfte in einer einmal eingenommenen Position verbleibt. Hierbei unterstützen auch zusätzlich Rastmittel, die nachfolgen noch näher beschrieben werden. Eine Skala auf der linearen Führungsbahn 24 erleichtert im Zusammenhang mit einer entsprechenden Markierung auf dem Schieber 25 ein Auslesen der aktuellen Einstellung des Rotationswinkels des Lagerbocks 7.

An dem Lagerbock 7 sind ferner Schwenkzapfen 27 angeordnet (zu beiden Seiten des Zapfenlochs 23, hier in der Figur jedoch nur einer der Zapfen mit Bezugsziffer versehen), über die der Schnittführungsblock 8 um die durch die Schwenkzapfen 27 gebildete Achse verschwenkbar mit entsprechenden Schwenklagern 28 geführt und gelagert ist. Die Ausrichtungsachse der Schwenkzapfen 27, die die Schwenkachse vorgibt, liegt dabei senkrecht zu der Richtung des Drehzapfens 22. Durch entsprechendes Verschwenken des Schnittführungsblocks 8 um die durch die Schwenkzapfen 27 vorgegebene Schwenkachse wird eine Einstellung des Slope-Winkels erreicht. Hierzu ist an dem Schnittführungsblock 8 fest angeformt eine zweite lineare Führung 29, über die ein Schieber 30 gleitend verschiebbar geführt ist, wobei auch dieser Schieber 30 ein G-förmiges Querschnittprofil aufweist und hinsichtlich seiner Dimensionierung in Bezug auf die Materialstärke sowie Materialwahl und im Verhältnis zu der im Wesentlichen quadratischen Querschnittsform der linearen Führung 29 so gewählt ist, dass er in einer einmal eingenommenen Position auf der linearen Führung 29 klemmend verbleibt, wobei hierbei zusätzliche Rastmittel unterstützen. An dem Schieber 30 ist an einer Wirkfläche ein Zapfen 31 angeformt, der in einer Führungsnut 32 an dem Lagerbock 7 aufgenommen und geführt ist. Die Führungsnut 32 hat einen analogen Verlauf wie eine gegenüberliegende Führungsnut 33, ist also ebenfalls gekrümmt und bildet zusammen mit dem Führungszapfen 31 eine Kulissenführung, die bewirkt, dass bei einem Verschieben des Schiebers 30 entlang der linearen Führung 29 ein Verschwenken des Schnittführungsblockes 8 um die durch die Ausrichtung der Schwenkzapfen 27 gebildete Schwenkachse erzielt wird.

In dieser Darstellung ist ferner ein in dem Schnittführungsblock 8 ausgebildeter Schnittführungsschlitz 34 zu erkennen, der der Führung eines Schneidwerkzeuges zum Setzen des Resektionsschnittes dient. Schließlich sind an der Oberseite des Schnittführungsblockes 8 angeordnete Aufnahmen 35 zu erkennen, die der Aufnahme eines Verbindungsstückes 36 zum Verbinden mit dem Kondylentaster 12 dienen. Durch die Positionierung der Aufnahmen 35 jeweils auf der rechten bzw. linken Seite des Schnittführungsblockes 8 kann der Kondylentaster 12 auf die innen liegende bzw. außen liegende Kondyle des jeweiligen Tibiaplateaus eingestellt werden, je nach dem, welche der beiden Kondylen stärker verschliessen ist und für die Höhenpositionierung als Referenz dient.

Erfindungswesentlich und die erhebliche Neuerung sind die Einstellmechanismen zum Einstellen der Varus/Valgus-Winkel und Slope-Winkel mit der erfindungsgemäßen Vorrichtung über die linearen Führungen 24 bzw. 29 und dem darauf bewegbaren Schiebern 25 bzw. 30, die im Zusammenwirken mit den gekrümmten Führungsnuten 26 bzw. 32 und den an den Schiebern 25, 30 angeordneten Führungszapfen 31 die Kulissenführung bewirken. Diese Art der Einstellmöglichkeit ist konstruktiv besonders kompakt und mit wenigen, einfach zu gestaltenden Teilen zu erreichen. Sie ist in ihrer einmal eingestellten Position besonders zuverlässig haltbar, was bei der Positionierung der Resektionsschnittebene durch entsprechendes Einstellen des Schnittführungsblockes 8 mit dem Schnittführungsschlitz 34 von erheblicher Wichtigkeit ist, um nicht etwa unter der Operation bei der Durchführung des Resektionsschnittes eine Positionsveränderung des Schnittführungsblockes 8 und damit des Schnittführungsschlitzes 34 zu erfahren.

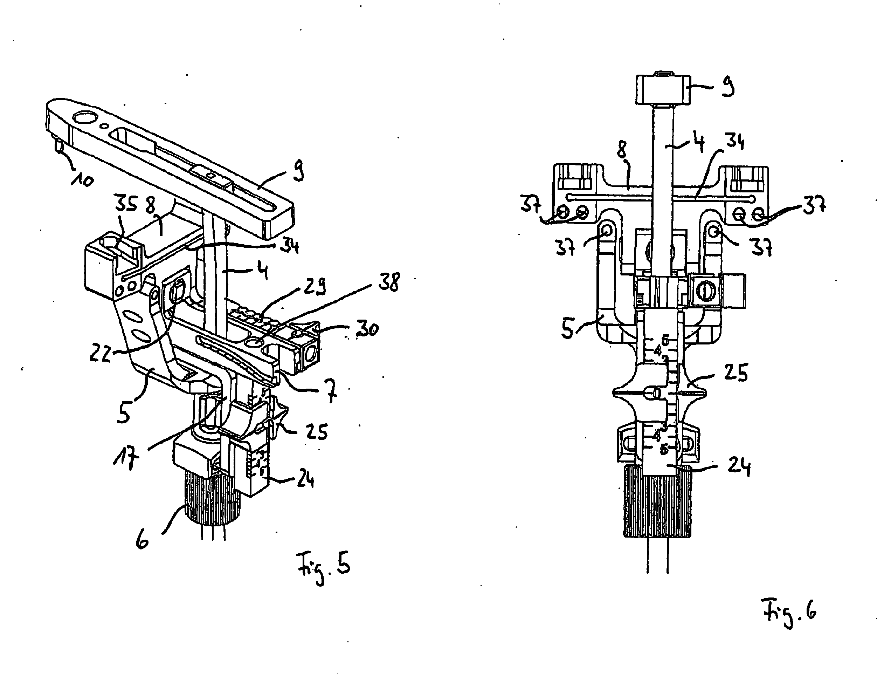

In Fig. 5 ist noch einmal eine Ansicht auf den oberen Abschnitt der Vorrichtung 1 (vgl. Fig. 1) im zusammengefügten Zustand dargestellt, wobei hier der Kondylentaster nicht gezeigt ist, dafür aber der am oberen Ende der Ausrichtstange 4 angeordnete Klemmbügel 9 mit dem Dorn 10. Gut zu erkennen ist hier, wie die beiden linearen Führungen 24, 29 mit den darauf angeordneten Schiebern 25 bzw. 30 nahe an und gegenüberliegend den jeweiligen Abschnitten mit den Führungsnuten der Lagerböcke 17 bzw. 7 liegen, um so ein Zusammenwirken der Kulissenführung und die entsprechende Winkeleinstellung zu ermöglichen.

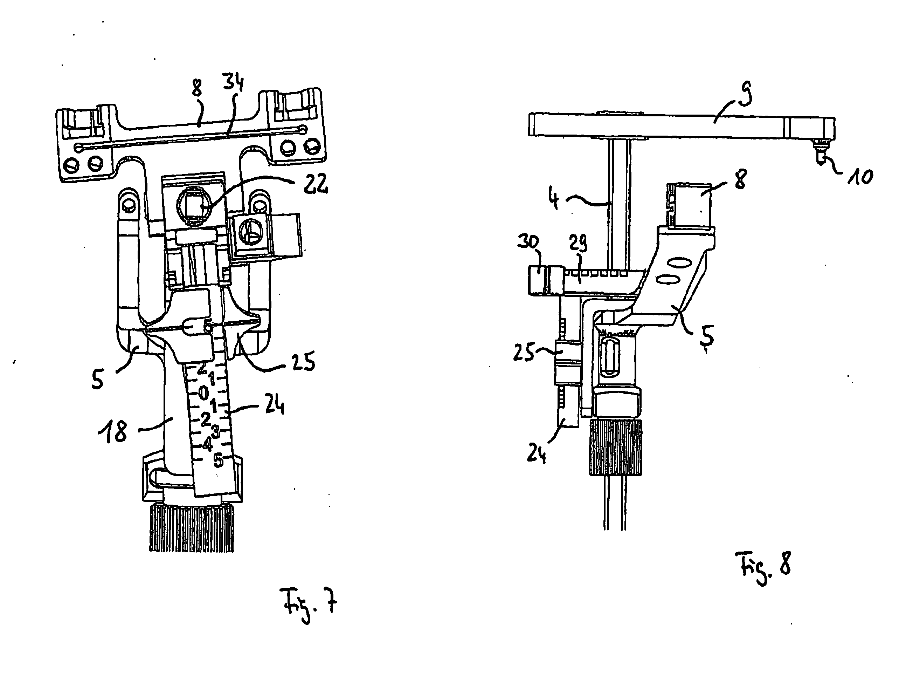

In den Figuren 6 und 7 ist - einmal mit dargestellter Ausrichtstange 4 und daran angeordnetem Klemmbügel 9 (in Fig. 6), einmal ohne diese Elemente - eine Ansicht des oberen Abschnittes der erfindungsgemäßen Vorrichtung gezeigt. In der in Fig. 6 ist der Varus/Valgus-Winkel bei 0° eingestellt. In Fig. 7 ist eine maximal nach links geneigte Einstellung gewählt. Hier kann gut gesehen werden, wie sich der Führungsblock 8 relativ zur Längsachse der Zapfenaufnahme am Basiskörper 5 neigt und damit auch den Schnittführungsschlitz 34 entsprechend zur Einstellung des Varus/Valgus-Winkels bewegt wird.

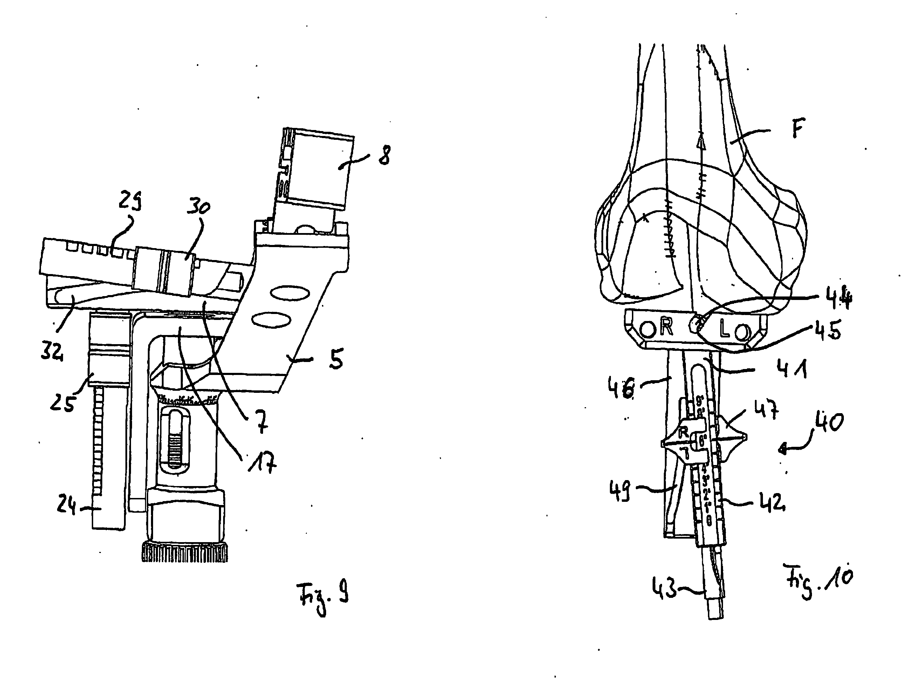

In den Figuren 8 und 9 sind zwei unterschiedliche Seitenansichten, in Fig. 8 mit Ausrichtstange 4 und daran angeordnetem Klemmbügel 9, in Fig. 9 ohne diese Elemente, die am oberen Ende der Vorrichtung angeordneten Teile gezeigt, einmal in einer Nulleinstellung für den Slope-Winkel, einmal in einer zum Einstellen des Slope-Winkels geneigten Position. In Fig. 9 ist nicht nur der Slope-Winkel durch Neigung bestimmt, sondern auch eine Einstellung des Varus/Valgus-Winkels vorgenommen, die von der Nulleinstellung abweicht, wie sie in Fig. 8 gezeigt ist. Gut zu erkennen ist in den beiden Figuren, dass der Schnittführungsblock gegenüber der Basis verkippt werden kann. In der Fig. 9 ist er nach rechts unten geneigt (um so den Slope-Winkel einzustellen) gezeigt.

Zu erkennen sind hier auch noch einmal die in den linearen Führungen 24 und 29 seitlich eingebrachten Rastnuten, in denen entsprechende im Inneren der Schieber liegenden Raststege bzw. Rastvorsprünge einrasten, zum Halten der Position. Darüber hinaus bieten diese Rastnuten ein taktiles Positionierungshilfsmittel, welches es dem Operateur erlaubt, ein Verstellen der Winkel abzuschätzen, indem hör- und fühlbare Einrastvorgänge gezählt und damit ein Verstellweg bzw. ein Winkel für die entsprechende Verstellung des Varus/Valgus- bzw. Slope-Winkels abgeschätzt wird.

Die Vorrichtung 1 (vgl. Fig. 1) kann mit einfachen Handgriffen in ihre Bestandteile zerlegt werden, wobei dies auch unter der Operation teilweise erfolgen kann. Dabei sind an der Basis wie auch am Schnittführungsblock 8 Pinlöcher 37 (vgl. Fig. 6) vorgesehen, durch die in bekannter Weise Befestigungspins geführt und in entsprechend angebrachte Bohrungen in dem Knochen gesteckt werden können. Ist der Basiskörper 5 mit entsprechenden Pins am Knochen festgelegt, so kann beispielsweise die Ausrichtstange 4 und kann der Klemmbügel 9 entfernt werden, und es kann auch die Fußklammer 2 entnommen werden. Ist daraufhin auch nach erfolgter Einstellung von der Schnitthöhe, Varus/Valgus-Winkel und Slope-Winkel der Schnittführungsblock 8 korrekt positioniert, können durch die entsprechenden Pinlöcher 37 an diesem Element Pins in zuvor gesetzte Bohrungen im Knochen geschoben und dort verankert werden, so dass der Schnittführungsblock 8 in seiner Ausrichtung festliegt. Dann können auch unter der Operation die weiteren Elemente inklusive des Lagerbocks 7 entfernt werden, so dass letztlich nur noch der Schnittführungsblock 8 am Knochen verbleibt, für das Setzen des Resektionsschnittes mithin besonders wenig Teile der erfindungsgemäßen Vorrichtung im Wege sind. Selbstverständlich kann der Operateur aber auch weitere Elemente der Vorrichtung beim Setzen des Resektionsschnittes im Aufbau belassen, bis hin zum vollständigen Aufbau inklusive der Fußklammer. Hier steht die Verwendungsform letztlich im Belieben des Operateurs.

Bei der Einstellung des Varus/Valgus-Winkels kann die durch eine Öffnung 38 (vgl. Fig. 5) in den Lagerbock 7 geführte Peilstange 11 den Operateur unterstützen, indem er mit dieser Peilstange 11 eine Ausrichtung in Bezug auf die Tibia- bzw. den Unterschenkel vornimmt.

Die erfindungsgemäße Vorrichtung 1 ist mit all ihren Elementen bevorzugt aus einem biokompatiblen Material, insbesondere Edelstahl gefertigt, und in der Formgebung so, dass eine einfache Reinigung und Sterilisation nach der OP für eine Wiederverwendung durchgeführt werden kann. Auch weist sie durch die Art der Konstruktion, insbesondere die Art der Gestaltung der Einstellmöglichkeiten für Varus/Valgus-Winkel und Slope-Winkel nur wenige und vergleichsweise groß dimensionierte Teile auf, so dass nicht etwa kleinteilige Schrauben oder dgl. zu desinfizieren und reinigen sind, schlimmstenfalls im Zuge der Operation verloren gehen und nach Versorgung der Wunde dort verbleiben können.

Die Verlegung sämtlicher Einstellmöglichkeiten, d.h. der Verstellmöglichkeiten sowohl des Varus/Valgus-Winkels als auch des Slope-Winkels sowie schließlich der Feinjustage hinsichtlich der Schnitthöhe an das obere Ende der Vorrichtung 1, also das Fortlassen einer Einstellung im Bereich der Fußklammer 2, wie es bei einigen Lösungen im Stand der Technik erfolgt, bietet weitere Vorteile hinsichtlich der Genauigkeit und auch Handhabbarkeit bei der Einstellung bzw. Vorgabe der Schnittebene für den Resektionsschnitt.

Schließlich ist noch anzumerken, dass eine Vorrichtung mit den im oberen Bereich an der Tibia T anzuordnenden Komponenten auch in einer intramedulären Befestigung verwendet werden kann, d.h. mit einer Festlegung an einem in das Tibia-Plateau in Richtung des Markkanals eingebrachten und dort befestigten Zapfen. Dann würde entsprechend anstelle des Klemmbügels 9 ein Haltebügel vorgesehen werden, der einerseits an dem in die Tibia eingebrachten Zapfen festgelegt ist, andererseits eine Ausrichtstange 4 aufweist. Bei einer solchen Festlegung kann ggf. auf eine Fußklammer 2 verzichtet werden, da häufig bei der intramedulären Festlegung bereits eine ausreichende Stabilität erreicht wird.

Im Nachfolgenden wird ein zweites Ausführungsbeispiel einer erfindungsgemäßen Vorrichtung zur Ausrichtung des Resektionsschnittes am unteren Femurplateau anhand der Figuren 10 bis 14 erläutert.

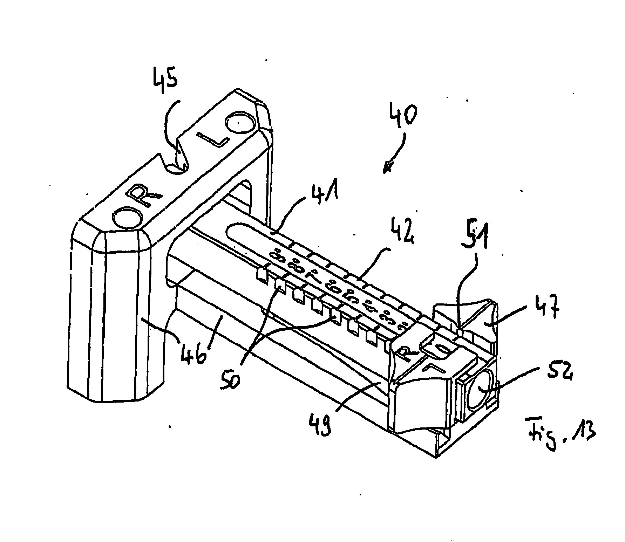

In Fig. 10 ist in schematischer Darstellung in einer Anordnung am unteren Plateau eines Femur F ein Ausrichtteil 40 einer erfindungsgemäßen Vorrichtung gemäß eines zweiten Ausführungsbeispiels gezeigt. Dieses Ausrichtteil 40 verfügt über einen Basiskörper 41, der in einem wesentlichen Abschnitt zugleich eine lineare Führungsbahn 42 bildet. In seinem Inneren ist der Basiskörper 41 mit einer durchgehenden Längsbohrung 52 (vgl. Fig. 13) versehen, durch die eine Anlagestange 43 hindurch ragt, die ortsfest an dem Femur F festgelegt ist in einer medialen Bohrung. Somit ist der Basiskörper 41 relativ zu dem Femur F ortsfest angeordnet. An seinem femurnahen Ende weist der Basiskörper 41 Schwenkzapfen 44 auf, mit welchem er in eine schlitzförmige Zapfenaufnahme 45 eines Lagerbocks 46 eingreift und demgegenüber verschwenkbar ist, bzw. den Lagerbock 46 darin verschwenkbar aufnimmt.

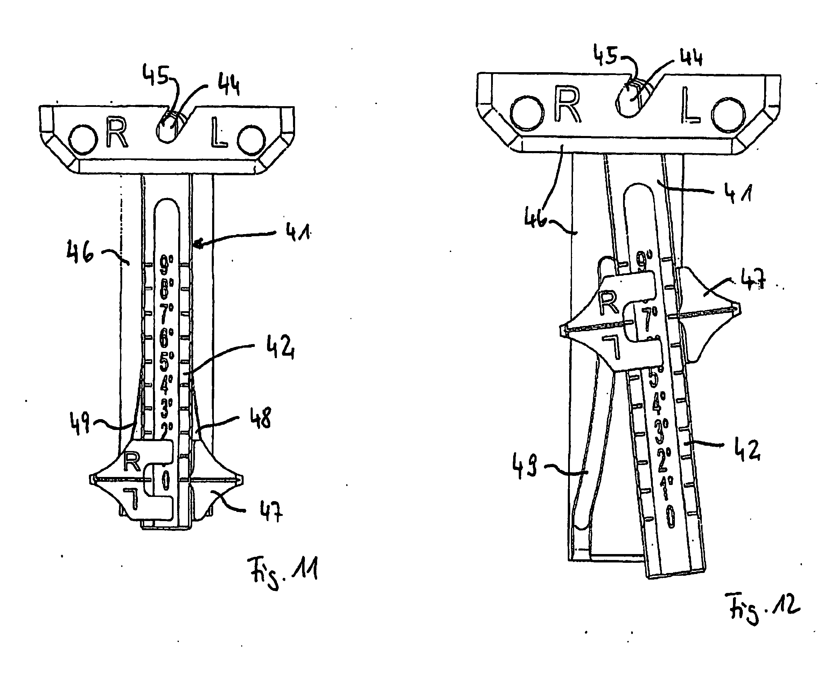

Auf der linearen Führungsbahn 42 ist ein Schieber 47 angeordnet, der vergleichbar den Schiebern 25 bzw. 30 im Ausführungsbeispiel zuvor mit G-förmigem Profil gebildet ist und so gestaltet, dass er auf der linearen Führungsbahn 42 mit Klemmsitz aufsitzen kann. Der Schieber 47 weist auf seiner hier nicht sichtbaren Rückseite in einem Wirkabschnitt einen Führungszapfen auf, mit dem er in einer in der Fig. 10 ebenfalls durch die lineare Führungsbahn 42 verdeckten Führungsnut sitzt und geführt ist. Diese Führungsnut ist auf einem Abschnitt des Lagerbocks 4 ausgebildet und verläuft spiegelbildlich zu der Längsachse dieses Abschnittes zu einer hier erkennbaren weiteren Führungsnut 49. Die Führungsnut 48, in der bei der in Fig. 10 dargestellten Ausrichtung der Schieber 47 mit seinem angeformten Zapfen läuft, ist in Fig. 11 zu erkennen.

Mit dem hier gezeigten Ausschnitt der Vorrichtung kann der Varus/Valgus-Winkel für die Bestimmung der Resektionsschnittebene am Femurplateau bestimmt werden. Dies funktioniert in gleicher Weise, wie die Einstellung der Winkel im oben beschriebenen ersten Ausführungsbeispiel. Auch hier läuft der am Schieber 47 angeformte Zapfen in der Führungsnut 48, die einen gekrümmten Verlauf hat. Dadurch ergibt sich im Zusammenspiel eine Kulissenführung, die für ein Verschwenken des Lagerbocks 46 gegenüber dem Basiskörper 41 um die durch den Schwenkzapfen 44 gebildete Schwenkachse bewirkt. Dadurch wird der Lagerbock 46 entsprechend eines zu wählenden Varus/Valgus-Winkels eingestellt. Der hier nicht dargestellte Schnittführungsblock wird über die im femurnahen Abschnitt des Lagerbocks 46 mit R und L bezeichneten Öffnungen (diese Bezeichnungen bestehen für rechtes bzw. linkes Bein, da diese Komponente für beide Anwendungsfälle verwendbar ist) angesetzt und mit entsprechenden Zapfen in diesen Öffnungen in einer Weise verbunden, dass er quer hierzu nicht mehr verrückt werden kann. Der hier nicht dargestellte Schnittführungsbock hat in der üblichen Weise einen Schnittführungsschlitz, der der Führung eines Schneidwerkzeuges beim Anbringen des Resektionsschnittes dient.

Zu erkennen ist auf der linearen Führungsbahn 42 eine aufgebrachte Skala mit Winkelangaben, die eine Abweichung des Varus/Valgus-Winkels von der 90°-Stellung aufzeigt. Auffällig ist hier auch, dass auf dem Schieber in spiegelverkehrter Lesart die Buchstaben "R" und "L" angeordnet sind. Dies bezeichnet wiederum die universale Anwendbarkeit der Vorrichtung. In der in Fig. 10 (auch in den weiteren Figuren) gezeigten Ausrichtung ist die Vorrichtung für eine Verwendung am rechten Bein eingestellt. Soll die Resektionsschnittebene am Femurplateau des linken Beines geplant werden, so ist hierfür der Schieber 47 durch Abziehen von der linearen Führungsbahn 42 zu lösen, so zu drehen, dass das L in einer Aufsicht gemäß Fig. 10 richtig herum und lesbar erscheint, und sodann wieder auf die lineare Führungsbahn 42 aufzuschieben. Dabei greift der rückseitig auf dem Schieber angeformte Führungszapfen nicht mehr in die Führungsnut 48, sondern in die spiegelverkehrt ausgebildete Führungsnut 49 auf der in Fig. 10 links dargestellten Seite des Lagerbocks. Auf diese Weise wird ein Verschwenken des Varus/Valgus-Winkels in die andere Richtung für die Einstellung am linken Bein ermöglicht.

Die auf der Skala angegebenen Gradzahlen geben hier tatsächliche gradmäßige Abweichungen aus der 90°-Stellung an, wofür die Linienführung der Führungsnuten 49 so berechnet und gewählt ist, dass bei einer äquidistanten Verschiebung des Schiebers 47 von einem Skalenteil zum nächsten jeweils eine Neigungsverstellung um 1 ° um die Schwenkzapfen 44 erfolgt und mithin eine Verstellung des Varus/Valgus-Winkels um jeweils 1°.

In den Figuren 11 und 12 ist das Ausrichtteil 40 einmal in einer Stellung für einen unverkippten Varus/Valgus-Winkel (90° zur Längsachse des Femur F) gezeigt, in Fig. 12 mit einer Abweichung bzw. Verkippung dieses Winkels um 7° aus dieser Normalstellung für das rechte Bein. Gut zu erkennen ist hierbei der Winkel, der zwischen dem Basiskörper 41 und dem Lagerbock 46, an dem der eigentliche Schnittführungsblock (nicht gezeigt) angelagert wird, eingenommen wird.

In Fig. 13 ist schließlich noch einmal in einer perspektivischen Ansicht das Ausrichtteil 40 gezeigt in der Normalstellung einer Ausrichtung des Varus/Valgus-Winkels senkrecht zur Längsachse des Femur F. In dieser Darstellung sind besonders gut zu erkennen die Rastkerben 50, die in äquidistanten Abständen entlang der linearen Führungsbahn 42 angeordnet sind, und in die entsprechende Vorsprünge 51 an dem Schieber 47 einrasten, wenn die jeweilige Position erreicht ist. Auch gut zu erkennen ist hier die zentrale Längsbohrung 52 im Basiskörper 41, durch die im Anwendungsfall die Anlagestange 43 (vergleiche Fig. 10) hindurchragt.

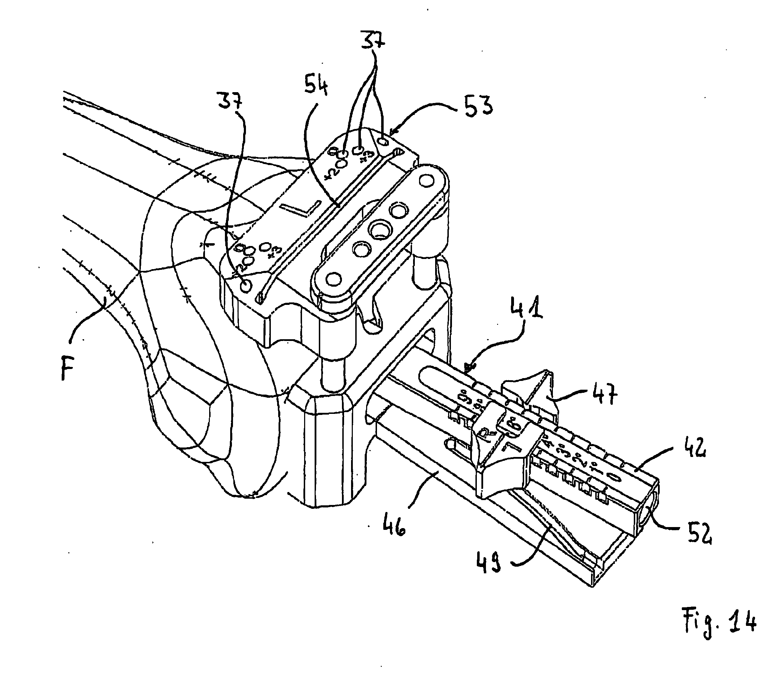

In Fig. 14 ist schließlich zu erkennen, wie an dem Lagerbock 46 an dessen an dem Femur F anliegenden, verdickten Ende ein Schnittführungsblock 53 angeordnet und mittels einer Loch-Stift-Befestigung festgelegt ist. Der Schnittführungsblock hat einen Schnittführungsschlitz 54 zum Führen der Klinge bzw. Schneide eines Schneidwerkzeuges. Ferner sind Pinlöcher 37 zu erkennen, durch die hindurch Befestigungspins in zuvor in den Femur F eingebrachte Bohrungen geführt werden können zum Festlegen des Schnittführungsblockes 53 am Femur F. Der Schnittführungsblock 53 wird typischerweise erst nach Einstellen des Varus-Valgus-Winkels angeordnet, kann aber auch bereits zuvor angebracht sein.

Auch dieses zweite Ausführungsbeispiel zeigt ein einfach aufgebautes Element, welches aus wenigen und vergleichsweise einfach zu handhabenden Teilen aufgebaut ist, die einfach voneinander gelöst werden können zu Zwecken der Reinigung und Sterilisation. Auch hier ist die präzise und auch unter Beanspruchung z.B. während eines Resektionsschnittes durch Vibrationen haltbare Einstellung der Winkellage der Resektionsschnittebene eingehalten. Sämtliche Elemente auch dieser Vorrichtung bestehen aus einem biokompatiblen Material, insbesondere Edelstahl.

Die vorstehend beschriebenen Ausführungsbeispiele sind nicht beschränkend, sie dienen lediglich der Erläuterung der Erfindung. Insbesondere ist die Erfindung nicht allein auf solche Anwendungen zur Planung und Positionierung der Resektionsschnittebene im Bereich des Femurplateaus bzw. Tibiaplateaus beschränkt. Hier können auch andere Resektionsschnittebenen an anderen Knochen geplant werden, z.B. am Humeruskopf. Dafür ist die Vorrichtung nur entsprechend anzupassen, wobei die Winkeleinstellung in erfindungsgemäßer Weise geschehen kann.

### Bezugszeichenliste

- 1: Vorrichtung für die Vorgabe einer Schnittebene für die Tibiaresektion
- 2: Fußklammer
- 3: Aufnahmerohr
- 4: Ausrichtstange
- 5: Basiskörper
- 6: Schraubhülse
- 7: Lagerbock
- 8: Schnittführungsblock
- 9: Klemmbügel
- 10: Dorn
- 11: Peilstab
- 12: Kondylentaster
- 13: Längsschlitz
- 14: Verbindungschraube
- 15: gekröpftes Vorderende
- 16: T-förmiges Verbindungsstück
- 17: Lagerbock
- 18: Hohlzapfen
- 19: Zapfenaufnahme
- 20: Peilstrich
- 21: Skala
- 22: Drehzapfen
- 23: Zapfenloch
- 24: lineare Führungsbahn
- 25: Schieber
- 26: Führungsnut
- 27: Schwenkzapfen
- 28: Schwenklager
- 29: lineare Führung
- 30: Schieber
- 31: Führungszapfen
- 32: Führungsnut
- 33: Führungsnut
- 34: Schnittführungsschlitz
- 35: Aufnahme
- 36: Verbindungsstück
- 37: Pinloch
- 38: Öffnung
- 40: Ausrichtteil
- 41: Basiskörper
- 42: lineare Führungsbahn
- 43: Anlagestange
- 44: Schwenkzapfen
- 45: Zapfenaufnahme
- 46: Lagerbock
- 47: Schieber
- 48: Führungsnut
- 49: Führungsnut
- 50: Rastkerbe
- 51: Rastvorsprung
- 52: Längsbohrung
- 53: Schnittführungsblock
- 54: Schnittführungsschlitz

- F: Femur
- P: Plateau
- T: Tibia

## Patentansprüche

1. Vorrichtung für die einstellbare Vorgabe einer Schnittebene für die Knochenresektion mit einem an dem Knochen (T; F) positionierbaren Basiskörper (5; 41) sowie einem an dem Basiskörper (5; 41) positionsveränderbar angelagerten eine Schnittführungsstruktur (34; 54) für das Führen eines Schneidwerkzeuges in der Schnittebene aufweisenden Schnittführungskörper (8; 53), wobei der Schnittführungskörper (8; 53) relativ zu dem Basiskörper (5; 41) mittels eines ersten verstellbaren Kippmechanismus um wenigstens eine erste Achse für eine Winkeleinstellung der Schnittebene verkippbar ist, wobei der erste Kippmechanismus eine an einem ersten Element der Vorrichtung angeordnete erste geradlinige Führung (24, 29; 42) und einen entlang der ersten geradlinigen Führung (24, 29; 42) in deren Längsrichtung linear verschiebbaren ersten Schieber (25, 30; 47) aufweist und wobei der erste Kippmechanismus ferner an einem relativ zu dem ersten Element um die erste Achse verkippbaren zweiten Element der Vorrichtung einen ersten Führungsabschnitt, gegenüber dem sich der erste Schieber (25, 30; 47) beim Verschieben mit einem Wirkabschnitt bewegt, aufweist, wobei in dem ersten Führungsabschnitt und dem ersten Wirkabschnitt zusammenwirkende Strukturen (26, 31, 32, 33; 48, 49) ausgebildet sind, die eine quer zu der Längsrichtung wirkende Kulissenführung ausbilden, **gekennzeichnet durch** eine gegenüber der Längsrichtung gekrümmte erste Führungsnut (26, 32, 33; 48, 49) oder einen solchen ersten Führungsschlitz in dem ersten Führungsabschnitt und einen in die erste Führungsnut (26, 32, 33; 48, 49) bzw. den ersten Führungsschlitz eingreifenden, darin geführten Führungsstift (31) bzw. Führungszapfen an dem Wirkabschnitt des ersten Schiebers (25, 30; 47).

2. Vorrichtung nach Anspruch 1, **gekennzeichnet durch** mit Rastmitteln (51) an dem ersten Schieber (47) zusammenwirkende Raststrukturen (52) im Bereich der ersten geradlinigen Führung (42), mittels derer der erste Schieber (47) in wenigsten zwei unterschiedlichen Positionen an der ersten Führung (42) **durch** Verrasten festlegbar ist.

3. Vorrichtung nach einem der vorhergehenden Ansprüche, **dadurch gekennzeichnet, dass** der erste Schieber(25, 30; 47) von der ersten geradlinigen Führung (24, 29; 42) lös- und abnehmbar an dieser angeordnet ist.

4. Vorrichtung nach einem der vorhergehenden Ansprüche, **dadurch gekennzeichnet, dass** die erste geradlinige Führung (24, 29; 42) eine im Querschnitt rechteckige, insbesondere quadratische, Führungsschiene ist und dass der erste Schieber (25, 30; 47) hülsenartig gebildet ist mit einer der Querschnittskontur der Führungsschiene entsprechenden, von einer Wandung bis auf eine durchgehende schlitzförmige Öffnung umschlossenen Innenkontur, wobei durch Wahl des Materials und der Wandstärke die schlitzförmigen Öffnung entgegen einer durch das Material ausgeübten Federkraft spreizbar ist.

5. Vorrichtung nach einem der vorhergehenden Ansprüche, **dadurch gekennzeichnet, dass** entlang der ersten geradlinigen Führung (24, 29; 42) eine Winkelskala und auf dem ersten Schieber (25, 30; 47) ein mit der Winkelskala zum Ablesen zusammenbringbarer Zeiger derart angeordnet sind, dass für eine bestimmte Position des ersten Schiebers (25, 30; 47) in der Längsrichtung durch die Zeigerstellung eine Winkelstellung der so um die erste Achse verkippten Schnittebene gegenüber einem vorgegebenen Referenzwinkel ablesbar ist.

6. Vorrichtung nach einem der vorhergehenden Ansprüche, **dadurch gekennzeichnet, dass** die Kulissenführung derart gestaltet ist, dass sich eine lineare Umsetzung der Wegstrecke, um die der erste Schieber (25, 30; 47) verlagert wird, zu einem Verkippungswinkel, um den die Schnittebene um die erste Achse verkippt wird, ergibt.

7. Vorrichtung nach einem der vorhergehenden Ansprüche, **dadurch gekennzeichnet, dass** der Schnittführungskörper (8) relativ zu dem Basiskörper (5) zusätzlich um eine zu der ersten Achse nicht parallele zweite Achse verkippbar ist und dass zum Verkippen um die zweite Achse ein unabhängig von dem ersten verstellbaren Kippmechanismus betätigbarer zweiter verstellbarer Kippmechanismus vorgesehen ist, wobei der zweite Kippmechanismus eine an einem ersten Element angeordnete zweite geradlinige Führung (24, 29) und einen entlang der zweiten Führung (24, 29) in deren Längsrichtung linear verschiebbaren zweiten Schieber (25, 30) aufweist und wobei der zweite Kippmechanismus ferner an einem relativ zu dem ersten Element um die zweite Achse verkippbaren zweiten Element einen zweiten Führungsabschnitt, gegenüber dem sich der zweite Schieber (25, 30) beim Verschieben mit einem zweiten Wirkabschnitt bewegt, aufweist, wobei in dem zweiten Führungsabschnitt und dem zweiten Wirkabschnitt zusammenwirkende Strukturen (26, 31, 32, 33) ausgebildet sind, die eine quer zu der Längsrichtung der zweiten Führung wirkende Kulissenführung ausbilden mit einer gegenüber der Längsrichtung gekrümmten zweiten Führungsnut (26, 32, 33) oder einem solchen zweiten Führungsschlitz in dem zweiten Führungsabschnitt und einem in die zweite Führungsnut (26, 32, 33) bzw. den zweiten Führungsschlitz eingreifenden, darin geführten zweiten Führungsstift (31) bzw. Führungszapfen an dem Wirkabschnitt des zweiten Schiebers (25, 30; 47).

8. Vorrichtung nach einem der vorhergehenden Ansprüche, **dadurch gekennzeichnet, dass** er eine Höhenverstelleinrichtung aufweist, mittels derer der Schnittführungskörper (8) relativ zu dem Basiskörper (5) in einer Höhenrichtung linear verlagerbar und in seinem Abstand zu diesem festlegbar ist.

9. Vorrichtung nach Anspruch 8, **dadurch gekennzeichnet, dass** in der Höhenverstelleinrichtung eine Stellschraube (6) zum Einstellen des Abstandes zwischen Basiskörper (5) und Schnittführungskörper (8) vorgesehen ist.

10. Vorrichtung nach einem der vorhergehenden Ansprüche, **dadurch gekennzeichnet, dass** sie für die Knochenresektion am proximalen Tibiaplateau eingerichtet ist.

11. Vorrichtung nach einem der vorhergehenden Ansprüche, **dadurch gekennzeichnet, dass** sie für die Knochenresektion am distalen Femurplateau eingerichtet ist.

## Claims

1. Device for the adjustable setting of a cutting plane for bone resection, with a base body (5; 41) that can be positioned on the bone (T; F), and with a cut-guiding body (8; 53) that is mounted so as to be variable in position on the base body (5; 41) and has a cut-guiding structure (34; 54) for guiding a cutting tool in the cutting plane, wherein the cut-guiding body (8; 53) is tiltable relative to the base body (5; 41) about at least a first axis by means of a first adjustable tilt mechanism in order to adjust the angle of the cutting plane, wherein the first tilt mechanism has a first rectilinear guide (24, 29; 42), arranged on a first element of the device, and a first slide (25, 30; 47) which is linearly displaceable along the first rectilinear guide (24, 29; 42) in the longitudinal direction thereof, and wherein the first tilt mechanism also has, on a second element of the device tiltable relative to the first element about the first axis, a first guide portion opposite which the first slide (25, 30; 47) moves with an active portion during the displacement, wherein interacting structures (26, 31, 32, 33; 48, 49) are formed in the first guide portion and in the first active portion and form a slotted guide acting transversely with respect to the longitudinal direction, **characterized by** a first guide groove (26, 32, 33; 48, 49) curved with respect to the longitudinal direction or such a first guide slit in the first guide portion, and a guide pin (31) engaging in the first guide groove (26, 32, 33; 48, 49) or the first guide slit and guided therein, on the active portion of the first slide (25, 30; 47).

2. Device according to Claim 1, **characterized by** locking structures (52) in the area of the first rectilinear guide (42), which interact with locking means (51) on the first slide (47) and by means of which the first slide (47) can be fixed by locking in at least two different positions on the first guide (42).

3. Device according to one of the preceding claims, **characterized in that** the first slide (25, 30; 47) is arranged on the first rectilinear guide (24, 29; 42) in such a way as to be releasable and detachable therefrom.

4. Device according to one of the preceding claims, **characterized in that** the first rectilinear guide (24, 29; 42) is a guide rail with a rectangular, in particular square cross section, and **in that** the first slide (25, 30; 47) is formed like a sleeve with an inner contour corresponding to the cross-sectional contour of the guide rail and enclosed by a wall, except for a continuous slitshaped opening, wherein, through the choice of the material and of the wall thickness, the slitshaped opening is expandable counter to a spring force exerted by the material.

5. Device according to one of the preceding claims, **characterized in that** an angle scale is arranged along the first rectilinear guide (24, 29; 42), and a pointer that can be brought together with the angle scale for reading off is arranged on the first slide (25, 30; 47), in such a way that, for a defined position of the first slide (25, 30; 47) in the longitudinal direction, the pointer position makes it possible to read off an angle position of the cutting plane thus tilted about the first axis in relation to a predefined reference angle.

6. Device according to one of the preceding claims, **characterized in that** the slotted guide is designed in such a way as to allow a linear conversion of the distance by which the first slide (25, 30; 47) is displaced to a tilt angle by which the cutting plane is tilted about the first axis.

7. Device according to one of the preceding claims, **characterized in that** the cut-guiding body (8) is tiltable relative to the base body (5) additionally about a second axis not parallel to the first axis, and **in that** the tilting about the second axis is permitted by provision of a second adjustable tilt mechanism that can be actuated independently of the first adjustable tilt mechanism, wherein the second tilt mechanism has a second rectilinear guide (24, 29), arranged on a first element, and a second slide (25, 30) which is linearly displeaceable along the second guide (24, 29) in the longitudinal direction thereof, and wherein the second tilt mechanism also has, on a second element tiltable relative to the first element about the second axis, a second guide portion opposite which the second slide (25, 30) moves with a second active portion during the displacement, wherein interacting structures (26, 31, 32, 33) are formed in the second guide portion and in the second active portion and form a slotted guide acting transversely with respect to the longitudinal direction of the second guide, with a second guide groove (26, 32, 33) curved with respect to the longitudinal direction or such a second guide slit in the second guide portion, and a second guide pin (31) engaging in the second guide groove (26, 32, 33) or the second guide slit and guided therein, on the active portion of the second slide (25, 30; 47).

8. Device according to one of the preceding claims, **characterized in that** it has a height adjustment mechanism by means of which the cut-guiding body (8) is linearly movable relative to the base body (5) in a vertical direction and can be fixed in its spacing from the latter.

9. Device according to Claim 8, **characterized in that**, in the height adjustment mechanism, a setting screw (6) is provided for adjusting the spacing between base body (5) and cut-guiding body (8).

10. Device according to one of the preceding claims, **characterized in that** it is configured for bone resection on the proximal tibial plateau.

11. Device according to one of the preceding claims, **characterized in that** it is configured for bone resection on the distal femoral plateau.

## Revendications

1. Dispositif destiné à prédéfinir de manière ajustable un plan de coupe pour la résection osseuse, comprenant un corps de base (5 ; 41) pouvant être positionné sur l'os (T ; F) ainsi qu'un corps de guidage de coupe (8 ; 53) appliqué en position variable sur le corps de base (5 ; 41) et présentant une structure de guidage de coupe (34 ; 54) pour guider un outil de coupe dans le plan de coupe, le corps de guidage de coupe (8 ; 53) pouvant être basculé par rapport au corps de base (5 ; 41) autour d'au moins un premier axe pour un ajustement angulaire du plan de coupe au moyen d'un premier mécanisme de basculement réglable, le premier mécanisme de basculement présentant un premier guide linéaire (24, 29 ; 42) disposé sur un premier élément du dispositif et un premier coulisseau (25, 30 ; 47) déplaçable linéairement le long du premier guide linéaire (24, 29 ; 42) dans sa direction longitudinale et le premier mécanisme de basculement présentant en outre, au niveau d'un deuxième élément du dispositif pouvant basculer par rapport au premier élément autour du premier axe, une première portion de guidage, par rapport à laquelle le premier coulisseau (25, 30 ; 47) se déplace avec une portion active lors du déplacement, des structures coopérantes (26, 31, 32, 33 ; 48, 49) étant réalisées dans la première portion de guidage et la première portion active, lesquelles constituent un guide à coulisse agissant transversalement à la direction longitudinale, **caractérisé par** une première rainure de guidage (26, 32, 33 ; 48, 49) courbée par rapport à la direction longitudinale ou une telle première fente de guidage dans la première portion de guidage et une goupille de guidage (31) ou un tourillon de guidage s'engageant dans la première rainure de guidage (26, 32, 33 ; 48, 49) ou dans la première fente de guidage et guidé(e) à l'intérieur de celle-ci, sur la portion active du premier coulisseau (25, 30 ; 47).

2. Dispositif selon la revendication 1, **caractérisé par** des structures d'encliquetage (52) coopérant avec des moyens d'encliquetage (51) sur le premier coulisseau (47) dans la région du premier guide linéaire (42), au moyen desquelles le premier coulisseau (47) peut être fixé par encliquetage dans au moins deux positions différentes sur le premier guide (42).

3. Dispositif selon l'une quelconque des revendications précédentes, **caractérisé en ce que** le premier coulisseau (25, 30 ; 47) est disposé sur le premier guide linéaire (24, 29 ; 42) de manière à pouvoir être détaché et enlevé de celui-ci.

4. Dispositif selon l'une quelconque des revendications précédentes, **caractérisé en ce que** le premier guide linéaire (24, 29 ; 42) est un rail de guidage de section transversale rectangulaire, en particulier quadrilatérale, et **en ce que** le premier coulisseau (25, 30 ; 47) est réalisé en forme de douille avec un contour interne correspondant au contour en section transversale du rail de guidage, entouré par une paroi à l'exception d'une ouverture traversante en forme de fente, l'ouverture en forme de fente pouvant être écartée à l'encontre d'une force de ressort exercée par le matériau, par le choix du matériau et de l'épaisseur de paroi.

5. Dispositif selon l'une quelconque des revendications précédentes, **caractérisé en ce qu'**une échelle angulaire est disposée le long du premier guide linéaire (24, 29 ; 42) et une aiguille pouvant être amenée en contact avec l'échelle angulaire pour effectuer une lecture est disposée sur le premier coulisseau (25, 30 ; 47) de telle sorte que pour une position déterminée du premier coulisseau (25, 30 ; 47) dans la direction longitudinale, la position de l'aiguille indique une position angulaire du plan de coupe ainsi basculé autour du premier axe par rapport à un angle de référence prédéfini.

6. Dispositif selon l'une quelconque des revendications précédentes, **caractérisé en ce que** le guide à coulisse est configuré de telle sorte que l'on obtienne une conversion linéaire de la distance de laquelle le premier coulisseau (25, 30 ; 47) est déplacé en un angle de basculement suivant lequel le plan de coupe est basculé autour du premier axe.

7. Dispositif selon l'une quelconque des revendications précédentes, **caractérisé en ce que** le corps de guidage de coupe (8) peut être basculé en outre par rapport au corps de base (5) autour d'un deuxième axe non parallèle au premier axe et **en ce que** pour le basculement autour du deuxième axe, il est prévu un deuxième mécanisme de basculement réglable pouvant être actionné indépendamment du premier mécanisme de basculement réglable, le deuxième mécanisme de basculement présentant un deuxième guide linéaire (24, 29) disposé sur un premier élément et un deuxième coulisseau (25, 30) déplaçable linéairement le long du deuxième guide (24, 29) dans sa direction longitudinale et le deuxième mécanisme de basculement présentant en outre, au niveau d'un deuxième élément pouvant basculer par rapport au premier élément autour du deuxième axe, une deuxième portion de guidage, par rapport à laquelle le deuxième coulisseau (25, 30) se déplace avec une deuxième portion active lors du déplacement, des structures coopérantes (26, 31, 32, 33) étant réalisées dans la deuxième portion de guidage et la deuxième portion active, lesquelles constituent un guide à coulisse agissant transversalement à la direction longitudinale du deuxième guide avec une deuxième rainure de guidage (26, 32, 33) courbée par rapport à la direction longitudinale ou une telle deuxième fente de guidage dans la deuxième portion de guidage et une deuxième goupille de guidage (31) au tourillon de guidage s'engageant dans la deuxième rainure de guidage (26, 32, 33) ou la deuxième fente de guidage et guidé(e) à l'intérieur de celle-ci, sur la portion active du deuxième coulisseau (25, 30 ; 47).

8. Dispositif selon l'une quelconque des revendications précédentes, **caractérisé en ce qu'**il présente un dispositif de réglage en hauteur au moyen duquel le corps de guidage de coupe (8) peut être déplacé linéairement par rapport au corps de base (5) dans une direction en hauteur et sa distance par rapport à celui-ci peut être fixée.

9. Dispositif selon la revendication 8, **caractérisé en ce que** dans le dispositif de réglage en hauteur est prévue une vis de réglage (6) pour ajuster la distance entre le corps de base (5) et le corps de guidage de coupe (8).

10. Dispositif selon l'une quelconque des revendications précédentes, **caractérisé en ce qu'**il est prévu pour une résection osseuse au niveau du plateau tibial proximal.

11. Dispositif selon l'une quelconque des revendications précédentes, **caractérisé en ce qu'**il est prévu pour une résection osseuse au niveau du plateau fémoral distal.
